# EUROPEAN PATENT SPECIFICATION

(11) **EP 4 206 330 B1**
(45) Date of publication and mention of the grant of the patent: **21.10.2026**
(21) Application number: 22216864.3
(22) Date of filing: 28.12.2022
(51) Int. Cl.: C12Q 1/6809, C12Q 1/6886

(54) **IDENTIFICATION SYSTEM AND KIT FOR DETECTION OF CIRCULATING BIOMARKERS OF CANCER**
IDENTIFIZIERUNGSSYSTEM UND KIT ZUM NACHWEIS VON ZIRKULIERENDEN BIOMARKERN VON KREBS
SYSTEME ET KIT D'IDENTIFICATION POUR LA DETECTION DE BIOMARQUEURS CIRCULANTS DU CANCER

(30) Priority: 28.12.2021 US 202163294359 P
(43) Date of publication of application: 05.07.2023
(73) Proprietor: Industrial Technology Research Institute, Chutung, Hsinchu 310401 (TW)
(72) Inventor: LI, Jian-Hao, 300 Hsinchu City (TW); HSIEH, Hui-Chu, 511 Shetou Township, Changhua County (TW); CHEN, Po-Chang, 300 Hsinchu City (TW); JIANG, Pei-Shin, 300 Hsinchu City (TW); LIN, Chih-Lung, 401 Taichung City (TW)
(74) Representative: Kurig, Thomas

(56) References cited:
- US-A1- 2018 104 187
- AICHUN LI ET AL: "Exosomal proteins as potential markers of tumor diagnosis", JOURNAL OF HEMATOLOGY & ONCOLOGY, vol. 10, no. 1, 1 December 2017 (2017-12-01), XP055771256, DOI: 10.1186/s13045-017-0542-8
- LOGOZZI MARIANTONIA ET AL: "Exosomes: A Source for New and Old Biomarkers in Cancer", CANCERS, vol. 12, no. 9, 9 September 2020 (2020-09-09), pages 2566, XP093044264, DOI: 10.3390/cancers12092566
- SAVVATEEVA E N ET AL: "Exosomal surface protein markers in diagnosis of colorectal cancer", MOLECULAR BIOLOGY : COVER-TO-COVER TRANSLATION = MOLEKULYARNAYA BIOLOGIYA, ACADEMY OF SCIENCES OF THE USSR, RU, vol. 51, no. 5, 13 October 2017 (2017-10-13), pages 659 - 665, XP036337875, ISSN: 0026-8933, [retrieved on 20171013], DOI: 10.1134/S0026893317050168
- VALLABHAJOSYULA PRASHANTH ET AL: "Tissue-specific exosome biomarkers for noninvasively monitoring immunologic rejection of transplanted tissue", THE JOURNAL OF CLINICAL INVESTIGATION, vol. 127, no. 4, 3 April 2017 (2017-04-03), GB, pages 1375 - 1391, XP093044268, ISSN: 0021-9738, DOI: 10.1172/JCI87993
- LIU HAN-MING ET AL: "Density distribution of gene expression profiles and evaluation of using maximal information coefficient to identify differentially expressed genes", PLOS ONE, vol. 14, no. 7, 17 July 2019 (2019-07-17), pages e0219551, XP093044359, DOI: 10.1371/journal.pone.0219551
- TIANYU WANG ET AL: "Comparative analysis of differential gene expression analysis tools for single-cell RNA sequencing data", BMC BIOINFORMATICS, BIOMED CENTRAL LTD, LONDON, UK, vol. 20, no. 1, 18 January 2019 (2019-01-18), pages 1 - 16, XP021269551, DOI: 10.1186/S12859-019-2599-6

## Description

### TECHNICAL FIELD

The disclosure relates to an identification system of circulating biomarkers and a development method of identifying circulating biomarkers, and more specifically, to an identification system of circulating biomarkers for cancer detection and a development method of identifying circulating biomarkers suitable for cancer detection.

### BACKGROUND

There is a great variety of circulating biomarkers, including DNA, mRNA, microRNA, metabolites and proteins, even circulating tumor cells and extracellular vesicles (EV) may be used as circulating biomarkers. These types of circulating biomarkers can come from minimally invasive sample types, including blood, saliva, urine, etc. The sample collection process is simple, which is suitable for disease screening or tracking.

Exosomes are lipid bilayer vesicles with a size of about 40 nanometers to 100 nanometers secreted by cells containing active molecules, and almost all types of cells (including cancer cells) secrete these vesicles. Exosomes and microvesicles are both extracellular vesicles. Microvesicles are formed by outward budding from the cell membrane, while exosomes are formed by inward budding of endosomes, and released via fusion of late endosomes with cell membrane. By secreting exosomes containing signaling molecules, cells can transmit information to adjacent cells or to distant cells or tissues through the circulatory system. When exosomes are absorbed by receiving cells, the carried signaling molecules such as proteins, mRNAs or microRNA will change the gene or protein expression of the receiving cell. A growing body of studies have shown that tumors can use exosomes to transmit signaling molecules to regulate the tumor microenvironment, and can also deliver exosomes to distant organs through the circulatory system, so that a metastatic environment suitable for colonization and growth of cancer cells is formed to promote tumor metastasis, which is called the pre-metastatic niche. Therefore, analyzing the composition of exosomes might be able to provide a convenient and accurate detection tool for the diagnosis and prognosis of breast cancer.

Circulating biomarker can be used for the diagnosis and monitoring of disease, but it is not easy to find suitable circulating biomarkers. There are roughly two approaches for the search of circulating biomarkers, one is to search for candidate biomarkers in the tissue and then verify their use in the circulatory system (such as blood), another is to directly screen biomarkers from the blood. The former approach can focus on markers directly related to the diseases, but the markers may not be good circulating biomarkers due to the interference of molecules from other tissues or cells in the blood. In contrast, when directly screening biomarkers from the blood, it is difficult to know if the biomarkers are directly related to the diseases because the composition of blood is complex, and the concentration of biomarkers from specific tissues may be low and blocked by other constituent molecules. Moreover, even if only considering the protein and nucleic acid biomarkers that have been observed on exosomes in the literature, the total number of genes corresponding to them exceeds 10,000.

Therefore, how to establish statistical analysis and machine learning algorithms to predict potential exosome biomarkers for subsequent cancer detection and recurrence model development from the large amount of various existing omics databases becomes increasingly important.
US 2018/104187 A1 discloses a method to identify circulating biomarkers suitable for cancer detection using exosomes for biomarker analysis for early detecting and characterizing of disease progression of cancer. Exosome expression markers associated to cancer are identified in cancer cell lines by exosome isolation and differential expression analysis; the markers may then be used to diagnose breast cancer using plasma samples directly.

### SUMMARY

The present invention is defined in appended claim 1. Beneficial embodiments are provided in the dependent claims. An example of the present disclosure provides an identification system of circulating biomarkers for cancer detection, a development method of circulating biomarkers for cancer detection, a cancer detection method and a kit. The identification system and the development method can predict potential exosome biomarkers for subsequent cancer detection and recurrence model development.

The identification system of circulating biomarkers for cancer detection of the example in the disclosure includes a) an identification module, b) a computing module and c) an evaluation module. The a) identification module is used to identify expression levels of multiple genes in normal tissue samples and tumor tissue samples, and select genes with high expression levels in the tumor tissue samples. The b) computing module uses tissue-specific genes and group-enriched genes to calculate a weight of each human tissue's contribution to plasma exosomes. The c) evaluation module compares gene expression levels of plasma exosomes of healthy people and cancer patients by using an overlapping index, and selects circulating biomarkers and combinations thereof suitable for detection and evaluation of the plasma exosomes.

The development method of circulating biomarkers for cancer detection of the example in the disclosure includes the following steps. Expression levels of multiple genes in normal tissue samples and tumor tissue samples are identified, and genes with high expression levels in the tumor tissue samples are selected. Afterwards, a weight of each human tissue's contribution to plasma exosomes is calculated using tissue-specific genes and group-enriched genes. Next, gene expression levels of plasma exosome of healthy people and cancer patients are compared by using an overlapping index, and circulating biomarkers and combinations thereof suitable for detection and evaluation of the plasma exosomes are then selected.

The identification system of circulating biomarkers for cancer detection of the example in the disclosure uses the aforementioned development method of circulating biomarkers for cancer detection.

The cancer detection method of the example in the disclosure uses the circulating biomarkers developed by the aforementioned identification system of circulating biomarkers for cancer detection, and the circulating biomarkers include BIRC5 and ART3.

The kit of the example in the disclosure uses the circulating biomarkers developed by the aforementioned identification system of circulating biomarkers for cancer detection, and the circulating biomarkers include BIRC5 and ART3.

Based on the above, the disclosure provides a development method of circulating biomarkers, the large amount of data in the protein or nucleic acid database of diseased tissues is analyzed by using methods such as the null hypothesis test and the overlapping index simulation, etc. The circulating biomarkers which can be used for disease diagnosis or monitoring are selected by simulating the changes of specific biomarkers in the circulatory system after the occurrence of the disease.

In order to make the above-mentioned features of the present disclosure more comprehensible, the following embodiments are given and described in detail with the accompanying drawings as follows.

### BRIEF DESCRIPTION OF THE DRAWINGS

FIG. 1 is a schematic diagram of the experiment and simulation results for the gene of calculated tissue weight, the horizontal axis represents the exosome RNA expression level (TPM, transcripts per million), and the vertical axis represents the probability density.
FIG. 2 is a schematic diagram showing the simulation results of tumor tissue highly expressed genes and overlapping index analysis, the horizontal axis represents the exosome RNA expression level (TPM, transcripts per million), and the vertical axis represents the probability density.
FIG. 3 is an analysis result diagram showing the verification results of breast cancer exosome biomarkers of ART3, BIRC5, CD274 and PTK7.
FIG. 4 is an analysis result diagram showing the results of the plasma interference test of plasma in exosomal ART3 and PTK7 analyses.
FIG. 5: (A) is an overlapping index simulation diagram of biomarker combination, in which the left diagram of (A) represents an overlapping index simulation diagram of a biomarker combination of BIRC5-ART3, and the right diagram of (A) represents an overlapping index simulation diagram of a biomarker combination of PTK7-ART3. (B) is a result diagram of performance comparison of biomarker pairs using the biomarker combinations of (A) to verify the combination through experiments.

### DETAILED DESCRIPTION OF DISCLOSURED EMBODIMENTS

The following examples are described in detail in conjunction with the accompanying drawings, but the provided examples are not intended to limit the scope of the present disclosure. Moreover, terms such as "include", "comprise", "have", etc. used in the text are all open-ended terms, that is, "including but not limited to".

The disclosure provides an identification system of circulating biomarkers for cancer detection and a development method of circulating biomarkers for cancer detection. The identification system of circulating biomarkers for cancer detection of the disclosure uses the development method of circulating biomarkers for cancer detection of the disclosure. Therefore, for the purpose of succinct description, the following mainly illustrates with the identification system of circulating biomarkers for cancer detection. The details of the development method of circulating biomarkers for cancer detection of the disclosure are basically repeated with the identification system of circulating biomarkers for cancer detection of the disclosure, so it will not be described in detail below.

The identification system of circulating biomarkers for cancer detection of the embodiment in the disclosure includes a) an identification module, b) a computing module and c) an evaluation module, wherein the identification module is used to select tumor tissue-upregulated gene markers, the computing module is used to calculate exosome weights of tissues, and the evaluation module is used to evaluate differences between healthy people and patients. In the following, the a) identification module, the b) computing module, and the c) evaluation module will be used to describe the identification system of the circulating biomarker for cancer detection according to an embodiment of the disclosure.

In terms of definition explanation, regarding the identification system of the circulating biomarker for cancer detection in the disclosure, wherein "identification system" includes hardware operating platforms (personal computers, supercomputers, etc.) and software (application programming interfaces, data processing algorithms, etc.), "module" can be a block, area, part, application area, or operation area in the identification system, but the disclosure is not limited thereto.

### a) Identification module

In the identification system of circulating biomarker for cancer detection disclosed in the disclosure, a) identification module compares normal tissue samples and tumor tissue samples in multiple genes covered by the exon-level RNA-seq or their products such as protein and mRNA expression levels, so as to select genes with high expression level in tumor tissue samples. Although the embodiment is mainly described with transcriptomics as an example, the disclosure is not limited thereto, and can also be applied to other physical data such as proteomics. It must be noted that before the genes with high expression level in the tumor tissue samples are selected, the data quality control/quality inspection (QC, quality control) of the physical data is performed first.

In the present embodiment, the genes with high expression level in tumor tissue samples are selected using statistical analysis methods, the statistical analysis methods include a null hypothesis test and a fold change threshold. In the following, the null hypothesis test and fold change threshold will be explained in detail.

### Null hypothesis test

In the present embodiment, the null hypothesis test is used to examine whether the average expression level of each gene in tumor tissue samples is significantly higher than that in normal samples. The null hypothesis test includes Welch's t-test, permutation test and false discovery rate (FDR). In more detail, the Welch's t-test is used to calculate a p value, the permutation test is used to adjust the p value, and then the false discovery rate is used as a standard for screening to reduce the probability of selecting false high-expression genes. In the following, the Welch's t-test, the permutation test and the false discovery rate will be explained in detail.

### Welch's t-test

In this embodiment, Welch's t-test allows tumor and normal tissue data variance to be different when testing whether the average expression level of each gene in tumor samples is significantly higher than that in normal samples. In more detail, the applied formula is as follows: $t = \frac{\overline{{X}_{T}} - \overline{{X}_{N}}}{\sqrt{\frac{{s}_{T}^{2}}{{N}_{T}} + \frac{{s}_{N}^{2}}{{N}_{N}}}}$
*̅X̅*̅_̅{̅T̅}̅ and ***̅X̅*̅_̅{̅N̅}̅:** Sample average gene expression level
***N*_{T}** and ***N*_{N}**: sample number
${s}_{T}^{2}$ and ${s}_{N}^{2}$**: Sample** variance

Test whether the average expression level (mean) of the gene of the tumor sample is statistically significantly higher than the average expression level of the gene of the normal sample, wherein ***̅X̅*̅_̅{̅T̅}̅ and *̅X̅*̅_̅{̅N̅}̅** are respectively the average gene expression level of tumor and normal samples, ***N*_{T}** and ***N*_{N}** are respectively the sample numbers of cancer and normal samples, and ${s}_{T}^{2}$ and ${s}_{N}^{2}$ are respectively the sample standard deviations of cancer and normal samples. The null hypothesis (*H₀*) here is: the average gene expression level of tumor samples ≤ the average gene expression level of normal samples, which belongs to the one-tailed test in the null hypothesis test. the probability threshold is set to be 0.5%, that is, if the probability of observing the current data statistically is less than 0.5% under the assumption condition of *H₀* (p value<0.005), then the hypothesis of *H₀* is rejected.

### Permutation test

When samples are limited, the resampling-based permutation test can improve the statistical power of t-test. In more detail, the applied formula is as follows: ${p}_{pm} = \frac{1}{{N}_{pm}} {\sum }_{j = 1}^{{N}_{pm}} I \left(p\leq {p}_{0}\right)$
*N*ₚₘ: **Number** of **random** permutations
*p*₀: **p-value** before permutation
${\sum }_{j = 1}^{{N}_{pm}} I \left(p\leq {p}_{0}\right)$:
Number of *p*≤*p₀* after random permutation
wherein ***N*ₚₘ** is a number of random permutations in the permutation test, ${\sum }_{j = 1}^{{N}_{pm}} I \left(p\leq {p}_{0}\right)$ is a cumulative number of ***N*ₚₘ** random permutations where p value ≤ p value before permutation , ***p*ₚₘ** is a p value calculated by the permutation test. In this embodiment, set *N*ₚₘ=10⁵, and *p*ₚₘ, can be regarded as a correction to the p value of Welch's t-test.

### False discovery rate

In this embodiment, when screening from a large number of genes, in order to reduce the incidence of false positives, the false discovery rate q≤0.005 is used as the standard. In more detail, the applied formula is as follows: $q = {N}_{gene} ∗ {p}_{n} / n$
***N*_{gene}:** Total number of screened genes
*p*ₙ: p-value for the n^{th} gene

In the process of screening a large number of genes at the same time, in order to reduce the probability of false positives, the p value obtained by the gene according to the null hypothesis test can be sorted from smallest to largest, and then the false discovery rate standard can be used to screen genes, wherein *N*_{gene} is the total number of screened genes, and *p*ₙ is the p value for the n^{th} gene (the genes have been sorted from smallest to largest according to the p value obtained by the null hypothesis test). After the maximum *n* value (*n*ₘₐₓ) satisfies q≤0.005 is calculated, the first*n*ₘₐₓ genes are the genes selected based on the false discovery rate.

### Fold change

Considering the interpretability of detection instrument results, this disclosure also sets appropriate fold change threshold conditions to exclude genes with too small fold change. The definition of fold change (FC) is: $FC = \frac{\overline{{X}_{T}}}{\overline{{X}_{N}}}$

That is, the ratio of the average gene expression level of tumor samples and normal samples. Although under the condition that the average gene expression level of tumor tissue is higher than the average expression level of normal tissue gene, a large number of genes can already be excluded (the exact number of genes excluded is related to the range of genes covered by each data set, and the data set used in this disclosure can exclude 40% to 50% of genes), and the number of genes left after screening with the condition of FC>2 is less than 5% of the original number of genes.

According to an embodiment of the present disclosure, exemplary operations are as follows. The triple-negative breast cancer RNA-seq gene expression level dataset GSE118527 in the Gene Expression Omnibus (GEO) database was analyzed, and the data of 88 cases with tumors and normal tissues around the tumors were compared, covering a total of 45,308 genes. The filter conditions are, for example:
(1) There are genes listed in ExoCarta, Vesiclepedia, or EVmiRNA exosome data sets, that is, genes that have been confirmed to appear in the exosome.
(2) The average gene expression level of tumor samples is higher than that of normal samples.
(3) Tumor tissue vs normal tissue satisfies q value < 0.005
(4) Fold change > 2

Among the 45308 genes, only 607 genes meet the above conditions, which greatly reduces the number of candidate genes.

Before b) computing module calculates the weight of each human tissue for plasma exosome contribution, the identification system of the circulating biomarker for cancer detection in this disclosure further refers to subcellular location information of exosome database to screen out the circulating biomarkers expressed on the surface or inside exosome. Circulating biomarkers expressed on the exosome surface can be further used for antibody binding.

### b) Computing module

In the identification system of circulating biomarker for cancer detection disclosed in the disclosure, b) computing module uses tissue-specific genes and group-enriched genes to calculate the weight of each human tissue for plasma exosome contribution. In more detail, the applied formula is as follows: ${Exo}_{gn} = {Tss 1}_{gn} * {C 1}_{gn} * {W 1}_{gn} + {Tss 2}_{gn} * {C 2}_{gn} * {W 2}_{gn} + …$
gn: Genes expressed in tissues and exosomes
Exo_{gn} : Expression level of exosomes in the gene in blood
Tss_{gn} : Tissue expression of the gene
C_{gn} : Multiplier of exosome expression released by tissue relative to tissue expression
W_{gn} :Tissue-released exosomes as a proportion of all exosomes in blood

Plasma exosomes are the sum of the exosomes secreted by various tissues/organs/blood cells in the blood. Therefore, the gene expression level of a gene (gn) on plasma exosomes can be expressed by the above formula. According to an embodiment of the disclosure, a total of 69 types of tissues, organs, or blood cells, etc. that provide detection data in large human omics databases such as HPA, FANTOM5, and GTEx are expected to cover all sources of exosomes in the blood (as shown in Table 1 below). In order to calculate the (*C*_{gn} = *W*_{gn}) weight of each tissue, according to an embodiment of the disclosure, human tissue and organ gene expression level data provided by online databases such as HPA, FANTOM5, and GTEx is used. First, several tissue-specific genes of each tissue are selected, the plasma exosome expression level of this type of gene is estimated as a contribution only from the highly expressed tissue, and then the (*C*_{gn} * *W*_{gn}) weight of the tissue is calculated. If there are no tissue-specific genes in a tissue, several group-enriched genes are selected, that is, a group of genes with significantly increased expression level in this tissue and other tissues, so as to jointly determine the (*C*_{gn} * *W*_{gn}) weight of each tissue.

**Table 1**

| Tissue/Organ | | | |
|---|---|---|---|
| breast | adipose tissue | skin | bone marrow |
| lymph node | lung | kidney | liver |
| gallbladder | spleen | stomach | duodenum |
| small intestine | rectum | colon | appendix |
| tongue | esophagus | smooth muscle | heart muscle |
| skeletal muscle | urinary bladder | retina | placenta |
| vagina | fallopian tube | cervix | endometrium |
| ovary | medulla oblongata | pons | thalamus |
| white matter | amygdala | hippocampal formation | midbrain |
| spinal cord | cerebellum | basal ganglia | choroid plexus |
| cerebral cortex | pituitary gland | hypothalamus | thyroid gland |
| parathyroid gland | tonsil | thymus | adrenal gland |
| pancreas | salivary gland | | |

| Blood cell | | | |
|---|---|---|---|
| platelet | NK-cell | naive CD8 T-cell | memory CD8 T-cell |
| naive CD4 T-cell | memory CD4 T-cell | T-reg | gdT-cell |
| MAIT T-cell | naive B-cell | memory B-cell | neutrophil |
| basophil | eosinophil | classical monocyte | non-classical monocyte |
| intermediate monocyte | myeloid DC | plasmacytoid DC | |

According to an embodiment of the disclosure, when calculating the weight, for each tissue-specific gene and group-enriched gene, HPA, FANTOM5 and GTEx human tissue expression level data are used to calculate the expression level probability density function of each tissue with the lognormal distribution for best fitting the expression level data of each tissue. After the expression level distribution of a tissue is obtained, it is used to calculate the gene expression level distribution of the exosome released into the blood by the tissue under different (*C*_{gn} * *W*_{gn}) weights.

According to an embodiment of the disclosure, the exosome gene expression level data of 149 healthy people are assembled, and for the selected tissue-specific genes and group-enriched genes, an in-house algorithm is used to adjust and test the weight of several tissues at the same time, and find out the (*C*_{gn} * *W*_{gn}) weight that can best restore the plasma exosome expression level distribution of all tissue-specific and group-enriched genes. The order of magnitude of tissue weight obtained by the simulation is as follows:
Fat: ~ 1 e-5
Breast: ~1e-5
Liver: ~1e-7
Lung: ~1e-3
Pancreas: ~1e-6
Skin: ~1e-5
Basophil: ~1e-5
Platelet: ~1e-1

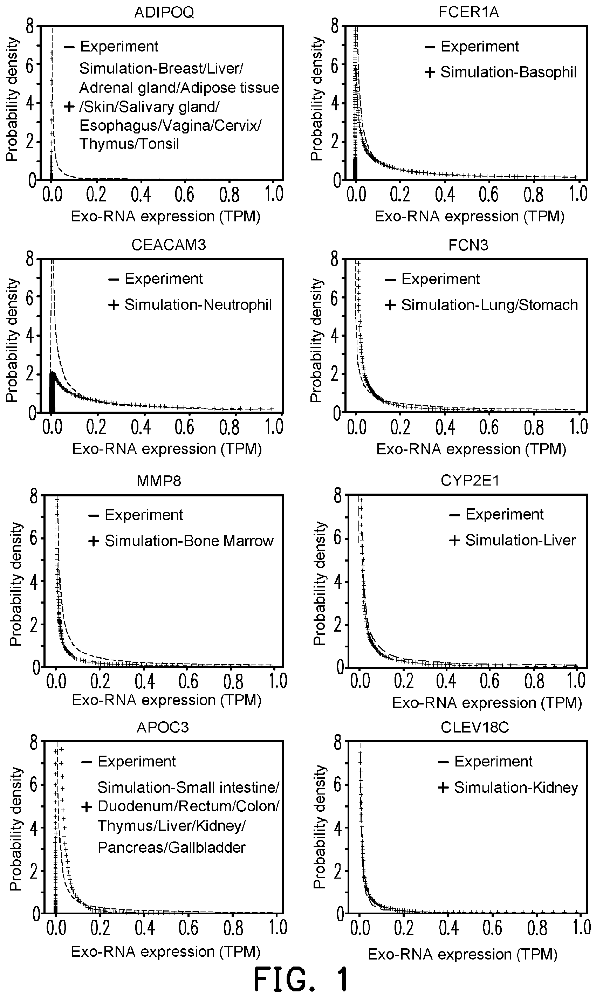

Please refer to FIG. 1, FIG. 1 is the schematic diagram of the experiment and simulation results for the gene of calculated tissue weight, wherein a large amount of human tissue expression level data of HPA, FANTOM5 and GTEx and the plasma exosome gene expression level data of 149 healthy people (GSE133684, GSE100206) are used to simulate the exosome expression level distribution probability density function of tissue-specific/group-enriched genes in the circulatory system, so as to calculate the weight of exosome released by different tissues. Taking basophil as an example, its expression level in FCER1A gene (simulation results shown in FIG. 1) is 80 times higher than the average expression level of other tissues in this gene, so FCER1A is classified as basophil tissue-specific gene, and is used together with other tissue-specific genes (10 in total) to simulate the weight of basophil exosome. Taking the lung as an example, in addition to using 5 lung tissue-specific genes (FCN3, AGER, SCGB3A2, SFTPD, SLC34A2, wherein FCN3 simulation results are shown in FIG. 1), 2 group-enriched genes (CLDN18 and GKN2) with significant expression levels in the stomach and lung are added to jointly simulate the exosome release weight of the lung.

### c) Evaluation module

In the identification system of circulating biomarker for cancer detection disclosed in the present disclosure, the c) evaluation module compares the gene expression levels of the plasma exosomes of healthy people and cancer patients by using an overlapping index, and selects circulating biomarkers and combinations thereof suitable for detection and evaluation of the plasma exosomes.

According to an embodiment of the present disclosure, the calculated weight is used to simulate a plasma exosome expression level distribution of circulating biomarker in the healthy people and the cancer patients after calculating the weight of each human tissue's contribution to plasma exosomes in the b) computing module. An intersection area of probability density functions of plasma exosome expression levels of the healthy people and the cancer patients are calculated according to the simulated plasma exosome expression level distributions, and the intersection area is the overlapping index. The smaller the intersection area (overlapping index), the better it is expected to be able to distinguish healthy and cancer statuses by plasma exosome detection. When the overlapping index is equal to 0.70 or less than 0.70, it is listed as a potential selection target. For example, the aforementioned overlapping index may be 0.70, 0.65, 0.60, 0.55, 0.50, 0.45, 0.40, 0.35, 0.30, 0.25, 0.20, 0.15 or 0.10, etc., but the present disclosure is not limited thereto. Furthermore, in addition to the overlapping index calculation, the biomarker selection also comprehensively considers the known characteristics of the gene, such as the gene function known in the literature, the subcellular locations of the gene product (protein) in the cell, and the plasma membrane confidence, etc., and finally the circulating biomarkers and combinations thereof suitable for detection and evaluation of the plasma exosomes are selected.

According to an embodiment of the present disclosure, after calculating the weight of the contribution of each tissue/organ/blood cell to the plasma exosome expression level, it is used to simulate the plasma exosome expression level distribution of healthy people in genes that have high expression level in the tumor tissue as identified in previous embodiment. Then, based on the gene expression level data of 88 cases of triple-negative breast cancer tissues from the GSE118527 data set and considering the phenomenon that cancer tissue cells release more exosomes than normal cells, the expression level distribution of individual genes in triple-negative breast cancer patients in plasma exosomes is simulated. According to the simulated plasma exosome expression level distributions of healthy and diseased people, the intersection area of plasma exosome expression level distributions of healthy and disease people for each individual gene can be calculated, and the overlapping index can be obtained. $η \left(T, N\right) = \int min \left[{f}_{T}\left(x\right),{f}_{N}\left(x\right)\right] dx .$

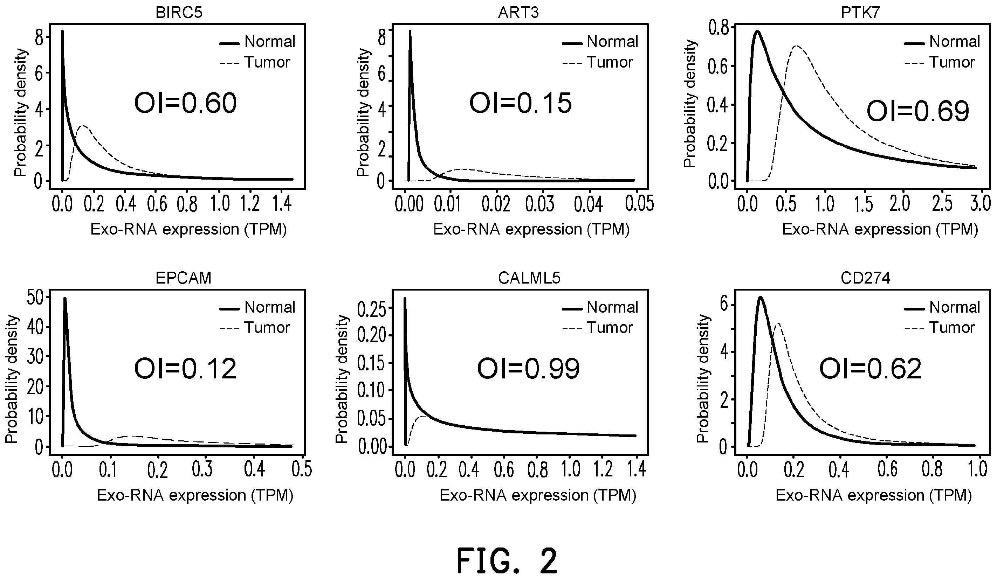

The smaller the overlapping index of a gene, the smaller the overlap of plasma exosome expression levels between breast cancer and breast cancer-free states of the gene, and thus potentially a better biomarker for distinguishing breast cancer and breast cancer-free states through exosome detection in the person to be examined. FIG. 2 is a schematic diagram showing the simulation results of tumor tissue-upregulated genes and overlapping index analysis. It can be seen in the figure that the logarithmic distribution graph is more complex than the normal distribution, while the overlapping index is not affected by the distribution type and can quantify the degree of intersection (similarity) of the two distributions.

### Validation of breast cancer exosomal biomarkers

According to the results of null hypothesis test and overlapping index analysis, the subcellular locations of these gene products (proteins) in the cell are further compared. The proteins noted to be expressed on the membrane in the HPA (human protein atlas) database are selected, or genes with plasma membrane confidence > 3 and extracellular confidence > 3 in the COMPARTMENTS Subcellular localization database are selected. From genes having fold change (FC) greater than 1.5, we sorted genes from low to high overlapping index, and ART3, BIRC5, CD274 and PTK7 are taken as examples for verification as exosome protein biomarkers. According to the annotations of HPA and COMPARTMENTS, ART3, BIRC5, CD274 and PTK7 may all be exosome surface proteins. In triple-negative breast cancer tissue RNA-seq study (GSE118527), through the analysis of a) identification module in this disclosure, the fold change and q values of ART3, BIRC5, CD274 and PTK7 are ART3: FC=2.7, q=2.3x10⁻⁹, BIRC5: FC=8.3, q=6.64x10⁻⁴⁵, CD274: FC =1.6, q=2.2x10⁻⁹ and PTK7: FC= 1.9, q=2.4x10⁻¹². In addition, according to the circulatory system simulation results of b) computing module in this disclosure, the amount and distribution of ART3 and BIRC5 in normal human plasma exosomes are significantly lower than those of CD274 and PTK7. In the verification of the cell line exosome, ultra-high-speed centrifugation is first carried out to separate cell line exosomes. After quantification by Nanoparticle Tracking Analysis (NTA), an equal number of exosomes are taken to compare the expression difference of these proteins in exosomes from normal breast epidermal cell lines (HMEC), triple-negative breast cancer cell lines (MDA-MB-231, MDA-MB-468 and HCC1806) and normal human plasma by immunoassay.

According to an example of the present disclosure, the processes of immunoassay include the following steps. Firstly, 96-well round bottom white plates carrying the magnetic beads which conjugated with ART3, BIRC5, CD274 and PTK7 antibodies are prepared. 100 µL of exosome samples isolated from different cell lines and plasma are added to the wells in a concentration of 5x10⁸ particles/mL, respectively. The reaction is performed on a shaker at 900 rpm at 37°C for 60 minutes under non-lysing conditions. After washing the magnetic beads with 0.1% Tween-PBST, we then add 100 µL of 0.5ug/mL biotin conjugated anti-CD81 antibody to each well and let the reactants in the well react for 60 minutes. After the magnetic beads are further washed with 0.1% Tween-PBST, 100 µL of streptavidin-HRP enzyme is added to each well and react for another 60 minutes. After the magnetic beads are washed with 0.1% Tween-PBST, the luminescent HRP substrate is added to react on the shaker for one minute and the luminescence signal is read.

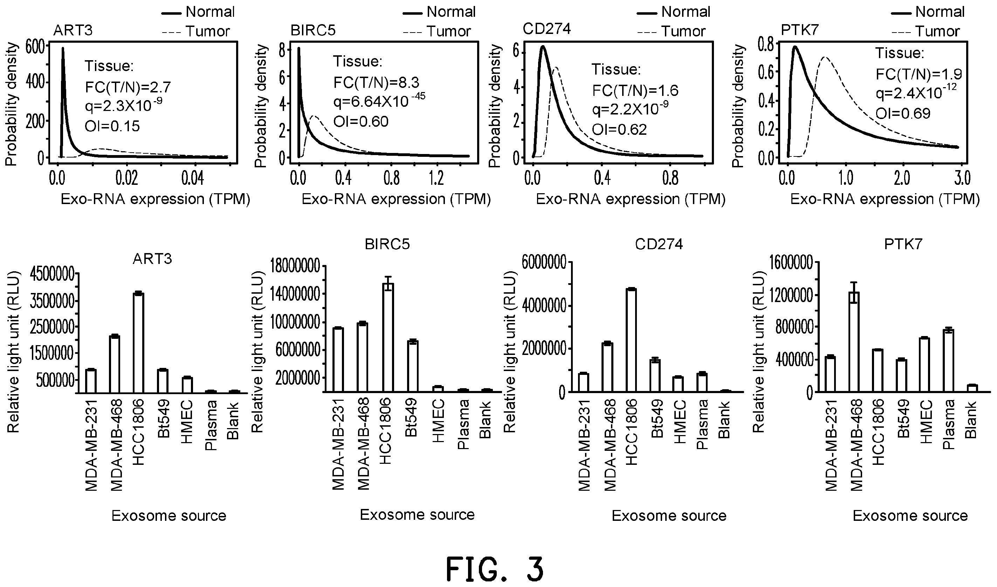

FIG. 3 is a verification result diagram of ART3, BIRC5, CD274 and PTK7, the candidates of breast cancer exosome biomarker. Please refer to FIG. 3, the results show that ART3, BIRC5, CD274 and PTK7 do exist in the exosome. BIRC5 demonstrates largest expression difference between the triple-negative cell line and the normal epidermal cell line, followed by ART3, and the difference of CD274 and PTK7 is not significant, which is consisted with the observation in the fold change in tissue. In addition, the expression levels of ART3 and BIRC5 in normal human plasma exosomes are much lower than in triple-negative cell line exosomes. This also indicates that, the lower expression levels of ART3 and BIRC5 in normal human plasma exosomes cause lesser interference to the tumor exosome by comparing with the expression levels of CD274 and PTK7, which is consistent with the simulation results under normal and tumor conditions.

### Plasma interference test

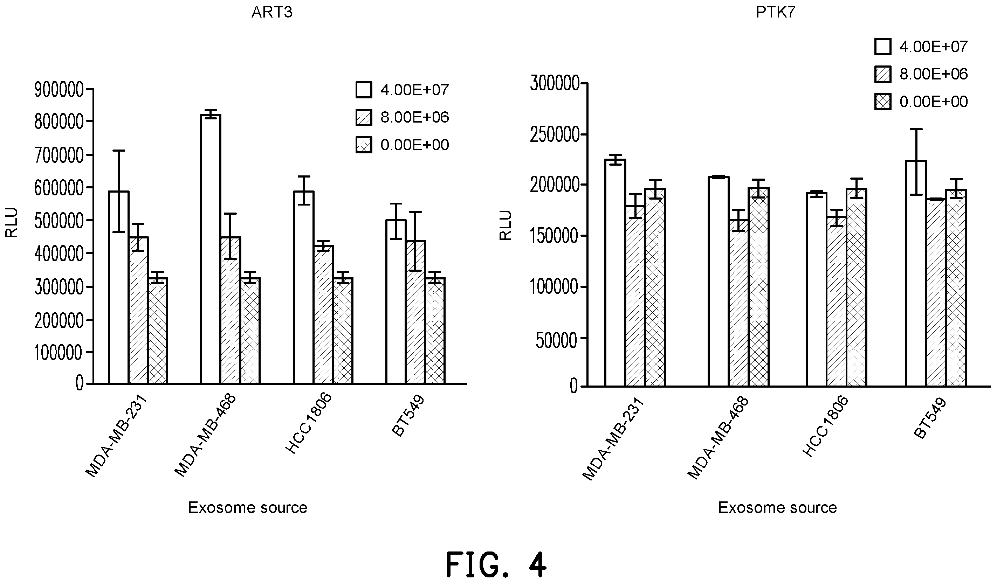

In this disclosure, the detecting sensitivity to breast cancer cell exosomes is evaluated by analyzing the samples spiked with various concentrations of breast cancer cell exosomes in plasma exosomes. According to an example of the present disclosure, exosomes from HCC1806, a triple-negative breast cancer cell line, are added to the 100 µL of size exclusion chromatography (SEC) processed plasma exosomes and make the final concentrations of HCC1806 exosomes to be 1x10⁹, 2x10⁸, 4x10⁷, and 8x10⁶ particles/mL respectively. Then the exosome surface protein is detected according to the above-mentioned magnetic bead immunoassay. FIG. 4 is the testing result diagram of plasma interference in ART3 and PTK7 immunoassay for exosome detection, wherein the left diagram in FIG. 4 demonstrates the plasma interference in ART3 immunoassay, and the right diagram in FIG. 4 demonstrates the plasma interference in PTK7 immunoassay. Please refer to FIG. 4, the results show that significant difference in signal would be observed even when as less of 8.0x10⁶ particles/mL of breast cancer cell line exosomes are added to the plasma exosomes which is in high concentration of 1.87x10¹⁰ particles/mL when exosomes are analyzed by ART3 immunoassay. In contrast, in the PTK7 immunoassay for exosome detection, there is no signal difference between the samples of plasma exosomes with and without 4x10⁷ or 8x10⁶ particles/mL of HCC1806 exosomes. This indicates that the interference caused by normal plasma is lesser in ART3 immunoassay than that of PTK7. When tumor exosome is in low concentration, exosome detection through PTK7 suffers more interference, and it is more difficult to distinguish the tumor sample from the normal. This experimental observation is consistent with the prediction of the overlapping index analysis.

### Selection of Capture-Detection combination for exosome detection

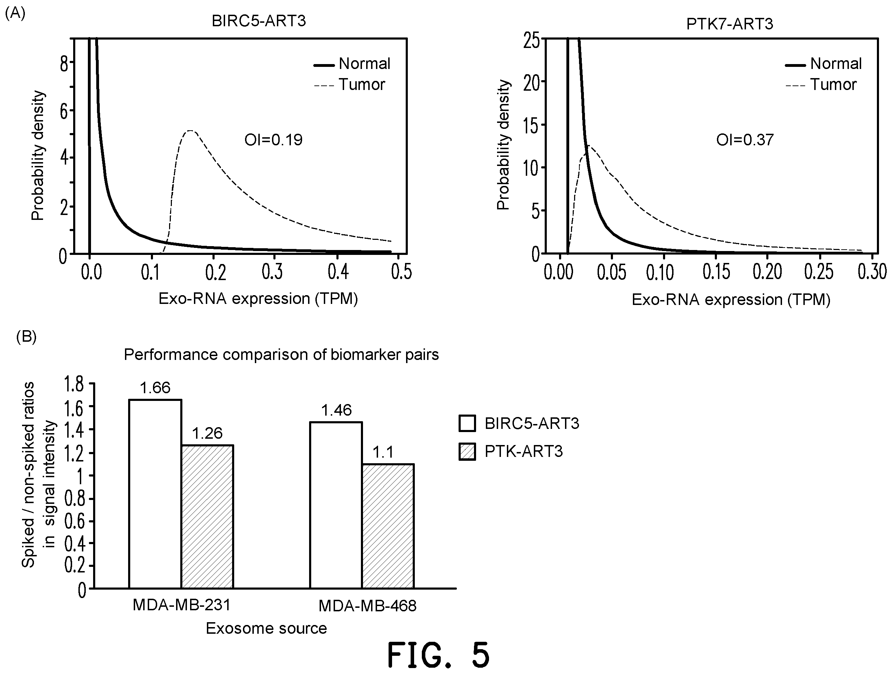

Exosomes are vesicles secreted by cells, which can carry molecules such as proteins, mRNA or microRNA of primitive cells. The exosomes of specific subgroups can be enriched by identifying surface proteins which perform an affinity purification, such as tumor exosomes, etc., and the biomarkers carried by it is further analyzed, so as to increase the specificity of detection. In the present disclosure, an optimized combination of exosome biomarkers can be developed by calculating C-D pair overlapping index of protein capture for enrichment and biomarker for detection. In an example of the present disclosure, BIRC5 and PTK7 are used respectively as surface proteins for affinity purification, and ART3 is used as a biomarker for detection. First, the expression level distributions of enrichment biomarkers (BIRC5 or PTK7) are used to simulate the proportion redistribution of exosomes from different tissue sources after the enrichment step, then the expression level distribution probability density function of the detection biomarker (ART3) of healthy and diseased people and the associated overlapping index are calculated. FIG. 5: (A) is an overlapping index simulation diagram of biomarker combination, the left diagram of (A) represents an overlapping index simulation diagram of a biomarker combination of BIRC5-ART3, and the right diagram of (A) represents an overlapping index simulation diagram of a biomarker combination of PTK7-ART3. (B) is the experimental verification result diagram. Please refer to (A) of FIG. 5, the overlapping indexes of BIRC5-ART3 and PTK7-ART3 combinations obtained by simulation are 0.19 and 0.37, respectively.

In one example of the present disclosure, the overlapping index of the biomarker combination is verified by evaluating their performance in immunodetection of tumor cell exosomes addition to the plasma. 800 µL plasma exosome separated by the size exclusion chromatography (SEC) is taken, and exosomes from MDA-MB-231 and MDA-MB-468, which are triple-negative breast cancer cell lines, are added and the concentrations of exosomes were made to be 1x10⁹ particles/mL for MDA-MB-231 and 3x10⁸ particles/mL for MDA-MB-468, respectively. Next, the performance in exosome detection of two C-D pairs, BIRC5-ART3 and PTK7-ART3, are compared according to the above-mentioned method of magnetic bead based immunoassay, wherein BIRC5 and PTK7 antibodies are used to capture exosomes, and ART3 antibodies are used as detection antibodies. Please refer to (B) of FIG. 5, it shows that the signal fold change of samples with and without the addition of tumor exosomes are 1.66-fold for MDA-MB-231 and 1.46-fold for MDA-MB-468 in the immunoassay utilizing BIRC5-ART3 combination, both results are better than those observed utilizing PTK7-ART3 combination, of which the signal differences are only 1.26-fold for MDA-MB-231 and 1.1-fold for MDA-MB-468. This observation is consistent with the trend predicted by the overlapping index.

This disclosure also provides a cancer detection method, using the circulating biomarker developed by the identification system of circulating biomarker for cancer detection described above. The circulating biomarkers include BIRC5 and ART3, which can be used to detect triple-negative breast cancer. For example, BIRC5 or ART3 antibodies are immobilized on carriers (such as magnetic beads or antibody-absorbable reaction disks) to capture exosomes in samples such as plasma, urine, and spinal fluid, and then antibodies which recognize BIRC5 or ART3 or other proteins are used for immunodetection. During the detection, enzymes such as horseradish peroxidase (HRP) and their substrates or fluorophore reagents can be used to generate signals for the detection of exosome biomarkers.

This disclosure also provides a kit, using the circulating biomarker developed by the identification system of circulating biomarker for cancer detection described above. The circulating biomarkers include BIRC5 and ART3, which can be used to detect triple-negative breast cancer. The kit contains BIRC5 or ART3 antibody or a solid support with BIRC5 or ART3 antibody, such as magnetic beads or a reaction plate that can absorb antibodies, or this antibody reagent is combined with antibodies which recognize BIRC5 or ART3 or other proteins, which is for exosome detection with or without reagents such as enzymes including HRP and their substrates or fluorophores.

In summary, the present disclosure provides an identification system of circulating biomarkers for cancer detection, a development method of circulating biomarkers for cancer detection, a cancer detection method and a kit. The identification system and development method use null hypothesis test, computational deconvolution, overlapping index and other methods, based on gene expression data of proteins and nucleic acids, identify genes whose gene expression level in tumor tissue is significantly higher than that in normal tissue, and consider the fold change of gene expression level of tumor tissue compared with gene expression level of normal tissue and other screening conditions. After that, the exosome expression level distribution of these genes in the blood of healthy and diseased people is simulated, combined with the exosome expression level distribution of healthy and diseased people after the enrichment step, so as to sort out the candidate biomarkers of proteins and nucleic acids, which are used as priority references for subsequent clinical specimen verification.

## Claims

1. A method to identify circulating biomarkers suitable for cancer detection, comprising:
measuring expression levels of multiple genes in normal tissue samples and tumor tissue samples, and selecting genes with higher expression levels in the tumor tissue samples than in normal tissue samples;
using tissue-specific genes and group-enriched genes to calculate a weight of each human tissue's contribution to plasma exosomes, wherein group-enriched genes are genes with significantly increased expression level in said tissue and other tissues; and
comparing expression levels of plasma exosome genes of healthy people and cancer patients by an overlapping index, wherein the overlapping index is the intersection area of probability density functions of plasma exosome expression levels of the healthy people and cancer patients, and selecting circulating biomarkers and combinations thereof suitable for detection and evaluation of the plasma exosomes.

2. The method according to claim 1, wherein a statistical analysis method is used to select the genes with high expression level in the tumor tissue samples, and the statistical analysis method includes a null hypothesis test and a fold change threshold, wherein the null hypothesis test includes:
using Welch's t-test to calculate a p value;
adjusting the p value by using a permutation test to improve the statistical power of t-test; and
performing a screening by using a false discovery rate as a criterium to reduce a probability of selecting false high-expression genes.

3. The method according to claim 1, further comprising comparing an exosome database and subcellular locations to screen out the circulating biomarkers expressed on the surface or inside exosomes before calculating the weight of each human tissue's contribution to plasma exosomes.

4. The method according to claim 1, further comprising using the calculated weight to simulate a plasma exosome expression level distribution of circulating biomarker in the healthy people and the cancer patients after calculating the weight of each human tissue's contribution to plasma exosomes.

5. The method according to claim 4, wherein the intersection area of the probability density functions of plasma exosome expression levels of the healthy people and the cancer patients are calculated according to the simulated plasma exosome expression level distributions, and the intersection area is the overlapping index.

6. The method according to claim 1, when the overlapping index of the plasma exosome gene is equal to 0.70 or less than 0.70, the plasma exosome gene is listed as a potential selection target.

7. An identification system of circulating biomarkers for cancer detection, wherein the identification system includes hardware operating platforms and software, the hardware operating platforms include computers, and wherein the software, when executed by the hardware operating platforms, is configured to perform steps in accordance with the method according to any one of claims 1 to 6.

## Patentansprüche

1. Verfahren zur Identifizierung zirkulierender Biomarker, geeignet für die Krebserkennung, umfassend:
Messen von Expressionsniveaus mehrerer Gene in normalen Gewebeproben und Tumorgewebeproben sowie das Auswählen von Genen, deren Expressionsniveau in den Tumorgewebeproben höher ist als in den normalen Gewebeproben;
Verwenden gewebespezifischer Gene und gruppenangereicherter Gene zur Berechnung eines Gewichts für den Beitrag jedes menschlichen Gewebes zu Plasma-Exosomen, wobei gruppenangereicherte Gene Gene sind, deren Expressionsniveau in dem genannten Gewebe und anderen Geweben signifikant erhöht ist; und
Vergleichen der Expressionsniveaus von Plasmaexosom-Genen bei gesunden Menschen und Krebspatienten mittels eines Überlappungsindex, wobei der Überlappungsindex der Schnittbereich der Wahrscheinlichkeitsdichtefunktionen der Plasmaexosom-Expressionsniveaus der gesunden Menschen und der Krebspatienten ist, und Auswählen von zirkulierenden Biomarkern und Kombinationen davon, die für den Nachweis und die Bewertung der Plasmaexosomen geeignet sind.

2. Verfahren nach Anspruch 1, wobei ein statistisches Analyseverfahren verwendet wird, um die Gene mit hohem Expressionsniveau in den Tumorgewebeproben auszuwählen, und das statistische Analyseverfahren einen Nullhypothesentest und einen Fold-Change-Schwellenwert enthält, wobei der Nullhypothesentest enthält:
Berechnen eines p-Werts mittels des Welch-t-Tests;
Anpassen des p-Werts unter Verwendung eines Permutationstests zur Verbesserung der statistischen Aussagekraft des t-Tests; und
Durchführen eines Screenings unter Verwendung einer Falsch-Entdeckungsrate als ein Kriterium, um die Wahrscheinlichkeit der Auswahl falsch identifizierter Gene mit hoher Expression zu verringern.

3. Verfahren nach Anspruch 1, ferner umfassend Abgleichen einer Exosom-Datenbank mit subzellulären Lokalisationen, um die zirkulierenden Biomarker herauszufiltern, die auf der Oberfläche oder im Inneren von Exosomen exprimiert werden, bevor das Gewicht des Beitrags jedes menschlichen Gewebes zu den Plasma-Exosomen berechnet wird.

4. Verfahren nach Anspruch 1, ferner umfassend Verwenden des berechneten Gewichts, um eine Verteilung der Plasma-Exosom-Expressionsniveaus zirkulierender Biomarker bei gesunden Personen und Krebspatienten zu simulieren, nachdem das Gewicht des Beitrags jedes menschlichen Gewebes zu den Plasma-Exosomen berechnet wurde.

5. Verfahren nach Anspruch 4, wobei der Schnittbereich der Wahrscheinlichkeitsdichtefunktionen der Plasma-Exosomen-Expressionsniveaus von gesunden Personen und Krebspatienten gemäß den simulierten Verteilungen der Plasma-Exosomen-Expressionsniveaus berechnet wird und der Schnittbereich den Überlappungsindex darstellt.

6. Verfahren nach Anspruch 1, wobei, wenn der Überlappungsindex des Plasma-Exosom-Gens gleich 0,70 oder kleiner als 0,70 ist, das Plasma-Exosom-Gen als potenzielles Auswahlziel aufgeführt wird.

7. Identifizierungssystem für zirkulierende Biomarker zur Krebserkennung, wobei das Identifizierungssystem Hardware-Betriebsplattformen und Software enthält, die Hardware-Betriebsplattformen Computer enthalten und wobei die Software, wenn sie von den Hardware-Betriebsplattformen ausgeführt wird, so konfiguriert ist, dass sie Schritte gemäß Verfahren nach irgendeinem der Ansprüche 1 bis 6 ausführt.

## Revendications

1. Un procédé d'identification de biomarqueurs circulants adaptés à la détection du cancer, comprenant :
le fait de mesurer des niveaux d'expression de plusieurs gènes dans des échantillons de tissus sains et des échantillons de tissus tumoraux, et le fait de sélectionner des gènes présentant des niveaux d'expression plus élevés dans les échantillons de tissus tumoraux que dans les échantillons de tissus sains ;
le fait d'utiliser des gènes spécifiques à un tissu et des gènes enrichis dans un groupe pour calculer le poids de la contribution de chaque tissu humain aux exosomes plasmatiques, les gènes enrichis dans un groupe étant des gènes dont le niveau d'expression est significativement accru dans ledit tissu et dans d'autres tissus ; et
le fait de comparer des niveaux d'expression de gènes des exosomes plasmatiques chez des personnes en bonne santé et chez des patients atteints d'un cancer au moyen d'un indice de chevauchement, l'indice de chevauchement étant l'aire d'intersection des fonctions de densité de probabilité des niveaux d'expression des exosomes plasmatiques chez les personnes en bonne santé et chez les patients atteints d'un cancer, et le fait de sélectionner des biomarqueurs circulants et des combinaisons de ceux-ci adaptés à la détection et à l'évaluation des exosomes plasmatiques.

2. Le procédé selon la revendication 1, dans lequel une méthode d'analyse statistique est utilisée pour sélectionner les gènes présentant un niveau d'expression élevé dans les échantillons de tissu tumoral, et le procédé d'analyse statistique comprend un test d'hypothèse nulle et un seuil de variation relative, le test d'hypothèse nulle comprenant :
le fait d'utiliser le test t de Welch pour calculer une valeur p ;
le fait d'ajuster la valeur p au moyen d'un test de permutation afin d'améliorer la puissance statistique du test t ; et
le fait de réaliser un criblage en utilisant un taux de fausses découvertes comme critère pour réduire la probabilité de sélectionner de faux gènes à forte expression.

3. Le procédé selon la revendication 1, comprenant en outre le fait de comparer une base de données d'exosomes et des localisations subcellulaires afin de passer au crible les biomarqueurs circulants exprimés à la surface ou à l'intérieur des exosomes avant de calculer le poids de la contribution de chaque tissu humain aux exosomes plasmatiques.

4. Le procédé selon la revendication 1, comprenant en outre le fait d'utiliser le poids calculé pour simuler une distribution des niveaux d'expression des biomarqueurs circulants dans les exosomes plasmatiques chez les personnes en bonne santé et les patients atteints d'un cancer, après avoir calculé le poids de la contribution de chaque tissu humain aux exosomes plasmatiques.

5. Le procédé selon la revendication 4, dans lequel la zone d'intersection des fonctions de densité de probabilité des niveaux d'expression des exosomes plasmatiques chez les personnes en bonne santé et chez les patients atteints d'un cancer est calculée en fonction des distributions simulées des niveaux d'expression des exosomes plasmatiques, et ladite zone d'intersection constitue l'indice de chevauchement.

6. Le procédé selon la revendication 1, dans lequel, lorsque l'indice de chevauchement du gène des exosomes plasmatiques est égal à 0,70 ou inférieur à 0,70, le gène des exosomes plasmatiques est répertorié comme cible de sélection potentielle.

7. Un système d'identification de biomarqueurs circulants pour la détection du cancer, dans lequel le système d'identification comprend des plateformes matérielles et un logiciel, les plateformes matérielles comprennent des ordinateurs, et dans lequel le logiciel, lorsqu'il est exécuté par les plateformes matérielles, est configuré pour mettre en œuvre les étapes conformes au procédé selon l'une quelconque des revendications 1 à 6.
